# EUROPEAN PATENT APPLICATION

(11) **EP 2 168 471 A2**
(43) Date of publication of application: **31.03.2010**
(21) Application number: 09012034.6
(22) Date of filing: 22.09.2009
(51) Int. Cl.: A61B 1/00

(54) **Flexible tube for endoscope and manufacturing method thereof**

(30) Priority: 25.09.2008 JP 2008245772
(71) Applicant: FUJIFILM Corporation, Minato-ku Tokyo (JP)
(72) Inventor: Takahashi, Nobuharu, Saitama-shi Saitama (JP)
(74) Representative: Klunker . Schmitt-Nilson . Hirsch

(57) **Abstract**

A flexible tube (21) is constituted of a flexible tubular structure (22), a jacket (23) for covering the periphery of the tubular structure, and a coating layer (24) for coating the jacket. The jacket has a soft resin layer (29) and hard resin layer (30), and has constant thickness throughout its length. The thickness of the soft resin layer is tapered from a distal end (22a) to a proximal end (22b) of the tubular structure. The hard resin layer applied on the soft resin layer gradually thickens from the distal end to the proximal end, conversely. While an assembly (34), which includes a plurality of tubular structures coupled in series, is passing through a head section (43), two layers of the soft resin layer and hard resin layer are successively formed thereon. The assembly with the jacket is wound up after cooling. After a dust removing step, a coating layer is applied on the hard resin layer.

## Description

### BACKGROUND OF THE INVENTION

### 1. Field of the Invention

The present invention relates to a flexible tube used in an insert section of an endoscope and a manufacturing method thereof.

### 2. Description Related to the Prior Art

An endoscope is known as medical equipment that has allowed an examination or surgery inside a living body cavity without incision. The endoscope has an insert section introduced into the human body cavity and a handling section provided on a proximal end of the insert section. The insert section is made of a slender flexible tube having a diameter of approximately 2 to 15 mm and a length of several tens of cm to 2 m.

The flexible tube is constituted of a flexible tubular structure, a jacket for covering the periphery of the structure, and a coating layer for coating the jacket. The flexible tubular structure is constituted of a helical coil, which is made of a metal ribbon wound helically, and a tubular net for surrounding the helical coil. The jacket includes a resin layer that is made of, for example, polyurethane resin, polyester resin or olefin resin. The coating layer is a thin resin layer. The coating layer is resistant to a chemical agent such as disinfectant.

To improve both the insertability of the insert section into the human body cavity and the operatability of the handling section, Japanese Patent Laid-Open Publication No. 63-249536 discloses a flexible tube that is soft at a distal end and hard at a proximal end. In this flexible tube, a hard resin layer is formed outside the flexible tubular structure, and a soft resin layer is formed outside the hard resin layer. Adjusting the thicknesses and distribution of the two resin layers varies the hardness of the flexible tube in a longitudinal direction. The coating layer is formed on the soft resin layer. It is known that the soft resin layer has a trouble called bleed in which plasticizer added to increase plasticity oozes from a surface thereof. The bleed occurring in the soft resin layer causes adhesion of dust to the gummy surface, and the adhering dust is hard to remove. The dust is removed in a dust removing step before forming the coating layer, and the dust removing step needs long time and a heavy load.

When the thicknesses of the hard resin layer and soft resin layer are almost the same, the volume of the inward hard resin layer is smaller than that of the outward soft resin layer. Accordingly, the hardness of the flexible tube is difficult to improve.

### SUMMARY OF THE INVENTION

An object of the present invention is to provide a flexible tube that reduces time and effort in a dust removing step for removing adhering dust, and a manufacturing method thereof.

Another object of the present invention is to provide the flexible tube having a jacket of two layers structure, for the purpose of improvement in hardness.

A flexible tube according to the present invention is constituted of a flexible tubular structure, a jacket for covering the periphery of the tubular structure, and a coating layer for coating the jacket. The jacket has a soft resin layer applied on the tubular structure and a hard resin layer applied on the soft resin layer. The flexible tube is used in an insert section of an endoscope.

The tubular structure has a first end and a second end. The soft resin layer is thicker at the first end of the tubular structure than at the second end thereof. The thickness of the soft resin layer varies continuously or in stages from the first end to the second end of the tubular structure.

The soft resin layer may include a thick section provided on the side of the first end, a thin section provided on the side of the second end, and a middle section provided between the thick section and the thin section. The thickness of the middle section continuously decreases from the thick section to the thin section.

The soft resin layer may include a thick section provided on the side of the first end, and a tapered section provided between the thick section and the second end. The thickness of the tapered section continuously decreases from the thick section to the second end.

The soft resin layer may include a thick section provided on the side of the first end, a medium-thickness section provided in the middle of the tubular structure, a connecting section provided between the thick section and the medium-thickness section, and a tapered section provided between the medium-thickness section and the second end. The connecting section connects the thick section to the medium-thickness section by continuously decreasing thickness. The thickness of the tapered section continuously decreases from the medium-thickness section to the second end.

The soft resin layer may include a thick section provided on the side of the first end, a medium-thickness section provided in the middle of the tubular structure, a thin section provided on the side of the second end, a first connecting section provided between the thick section and the medium-thickness section, and a second connecting section provided between the medium-thickness section and the thin section. The first connecting section connects the thick section to the medium-thickness section by continuously decreasing thickness. The second connecting section connects the medium-thickness section to the thin section by continuously decreasing thickness.

The thickness of the soft resin layer continuously decreases from the first end to the second end, and the thickness of the hard resin layer continuously increases from the first end to the second end. The outside diameter of the jacket for covering the tubular structure is substantially constant throughout its length.

The thickness of the soft resin layer decreases in stages from the first end to the second end, and the thickness of the hard resin layer increases in stages from the first end to the second end. The outside diameter of the jacket for covering the tubular structure is substantially constant throughout its length.

A method for manufacturing a flexible tube has the steps of feeding a flexible tubular structure into a head section and passing the tubular structure through the head section, extruding soft resin in the head section to form a soft resin layer on the periphery of the tubular structure, extruding hard resin in the head section to form a hard resin layer on the soft resin layer, adjusting extrusion pressure of the soft resin and hard resin in accordance with the thickness of said soft resin layer and hard resin layer, cooling the tubular structure with a jacket, and winding up the tubular structure with the jacket.

According to the present invention, since the hard resin layer constitutes an outer layer of the jacket, bleed due to plasticizer hardly occurs. Thus, dust is less likely to adhere to the hard resin layer, and adhering dust is easily removable. Outwardly providing the hard resin layer allows increase in the volume of the hard resin layer. Therefore, it is possible to improve the hardness of the flexible tube without use of a hardening resin.

### BRIEF DESCRIPTION OF THE DRAWINGS

For more complete understanding of the present invention, and the advantage thereof, reference is now made to the following descriptions taken in conjunction with the accompanying drawings, in which:
Fig. 1 is a schematic view of an endoscope;
Fig. 2 is a cross sectional view of a flexible tube in which the thickness of a soft resin layer is tapered to its proximal end;
Fig. 3 is a flowchart of a tubular structure covering process;
Fig. 4 is an explanatory view of a joint member for coupling flexible tube assemblies;
Fig. 5 is a block diagram showing the schematic structure of an extrusion apparatus;
Fig. 6 is an explanatory view that schematically shows variations in the thicknesses of a hard resin layer and soft resin layer in covering coupled assemblies with a jacket;
Fig. 7 is a graph showing variation in assembly feeding speed in applying the jacket;
Fig. 8 is a cross sectional view of a flexible tube according to another embodiment;
Fig. 9 is a cross sectional view of a flexible tube according to further another embodiment;
Fig. 10 is a cross sectional view of a flexible tube according to further another embodiment; and
Fig. 11 is a cross sectional view of a flexible tube according to further another embodiment.

### DESCRIPTION OF THE PREFERRED EMBODIMENTS

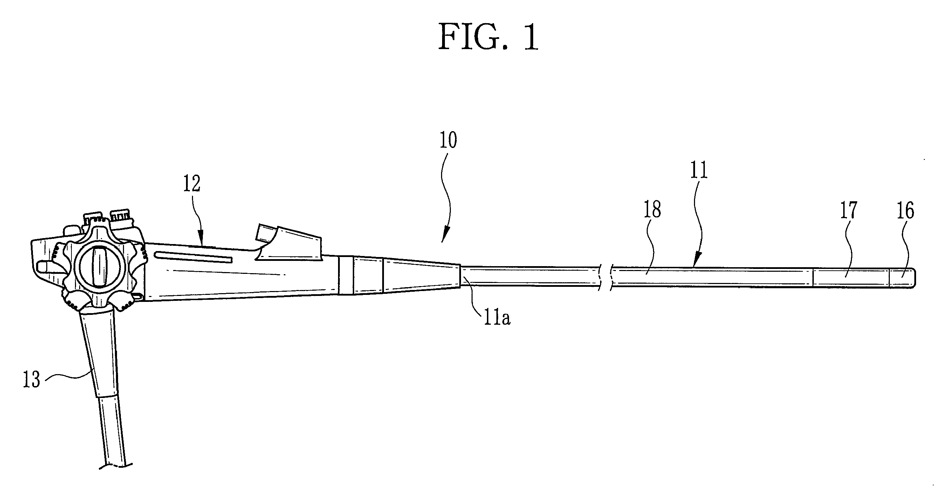

As shown in Fig. 1, an endoscope 10 is constituted of an insert section 11 to be introduced into a human body cavity, a handling section 12, and a universal cord 13. A base end 11a of the insert section 11 is attached to the handling section 12. The universal cord 13 attached to the handling section 12 is detachably connected to an external processor device (not illustrated) via a connector.

The insert section 11 has a distal portion 16, a bending portion 17, and a flexible portion 18 disposed in this order from its distal end. The distal portion 16 contains an imaging sensor (not illustrated) for capturing an image of an internal body part. The bending portion 17 flexibly bends inside the body cavity, and aims the distal portion 16 at a desired direction. The flexible portion 18 provided between the bending portion 17 and the handling section 12 has a length of, for example, 1.3 m to 1.7 m, and occupies most of the insert section 11.

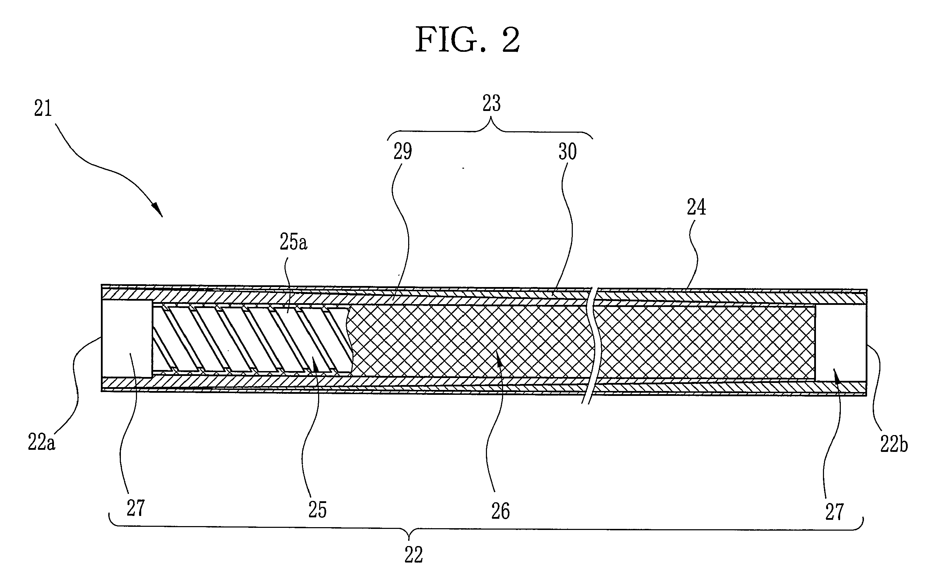

The flexible portion 18 is constituted of a flexible tube 21 shown in Fig. 2. The flexible tube 21 includes a flexible tubular structure 22, a jacket 23 or covering layer for covering the periphery of the tubular structure 22, and a coating layer 24 for coating the jacket 23. A proximal end 22b of the tubular structure 22 is attached to the handling section 12, and a distal end 22a thereof is attached to the bending portion 17. The tubular structure 22 is constituted of a helical coil 25, a tubular net 26 for covering the periphery of the helical coil 25, and end fittings 27 or rings of metal fitted into both ends of the tubular structure 22. The helical coil 25 consists of a metal ribbon 25a wound helically into a tubular shape. The tubular net 26 is made of thin metal wires braided into a tubular shape.

The jacket 23 has a soft resin layer 29 formed around the tubular structure 22 and a hard resin layer 30 formed on the soft resin layer 29. The soft resin layer 29 and hard resin layer 30 are made of thermoplastic elastomers the hardness of which is different from each other. The thermoplastic elastomers include, for example, a polyurethane resin, polyester resin or polyolefin. The coating layer 24 is made of a fluorocarbon polymer, silicone resin or polyurethane resin, and imparts resistance to a chemical agent such as disinfectant. In Fig. 2, the jacket 23 and coating layer 24 are illustrated thicker relative to the diameter of the tubular structure 22, for the purpose of clearly showing layer structure.

The thickness of the soft resin layer 29 is tapered from the distal end 22a to the proximal end 22b of the tubular structure 22. The hard resin layer 30, in contrast to the soft resin layer 29, becomes thicker as it goes from the distal end 22a to the proximal end 22b. The total sum of the thicknesses of the soft resin layer 29 and hard resin layer 30, that is, the thickness of the jacket 23 is constant throughout the entire length of the tubular structure 22, so that the flexible tube 21 has a constant diameter.

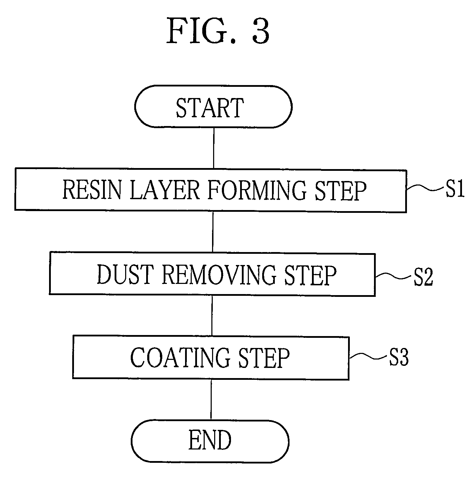

Next, a tubular structure covering process for forming the jacket 23 and coating layer 24 on the periphery of the tubular structure 22 will be described. As shown in Fig. 3, the tubular structure covering process has a resin layer forming step S1, a dust removing step S2 and a coating step S3.

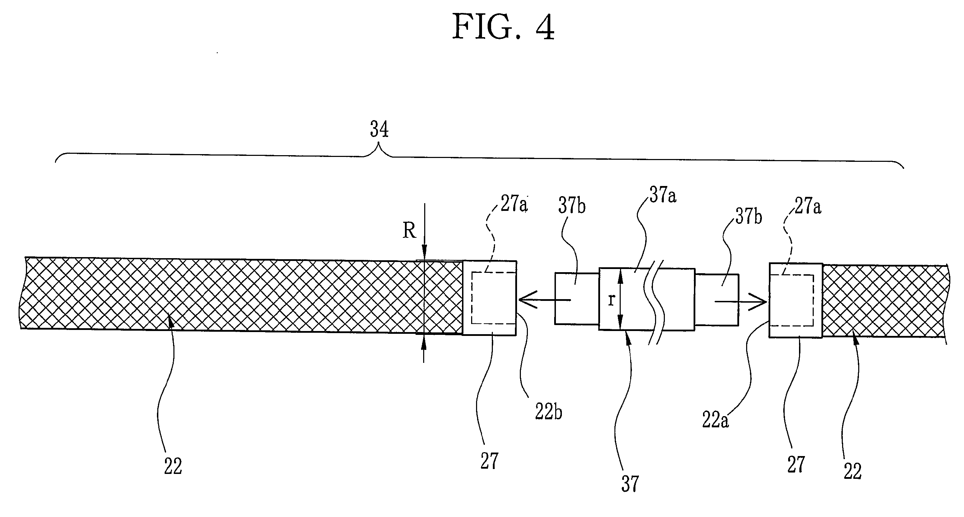

In the resin layer forming step S1, as shown in Fig. 4, the jacket 23 is formed on the periphery of an assembly 34 that includes a plurality of tubular structures 22 coupled in series. The adjoining two tubular structures 22 are coupled by a joint member 37. The joint member 37 has a main body 37a and joining portions 37b provided on both sides of the main body 37a. By tightly fitting each joining portion 37b into an inner surface 27a of the end fitting 27, the joint member 37 joins the distal end 22a of the tubular structure 22 to the proximal end 22b of another tubular structure 22. The outside diameter "r" of the main body 37a of the joint member 37 is smaller than the outside diameter "R" of the tubular structure 22.

The surface of the joint member 37 is coated with a separating material or releasing agent such as Teflon (trademark). Thus, the jacket 23 on the periphery of the joint member 37 is easy to peel off, after being formed on the assembly 34. The main body 37a of the joint member 37 is flexible. As will be described later in detail, when the jacket 23 is continuously formed on the assembly 34, the thickness ratio between the soft resin layer 29 and hard resin layer 30 gradually varies on the tubular structure 22, and is reset to an initial value on the main body 37a. Accordingly, the length of the main body 37a is determined in consideration of feeding speed of the assembly 34 and variation in extrusion pressure of resin.

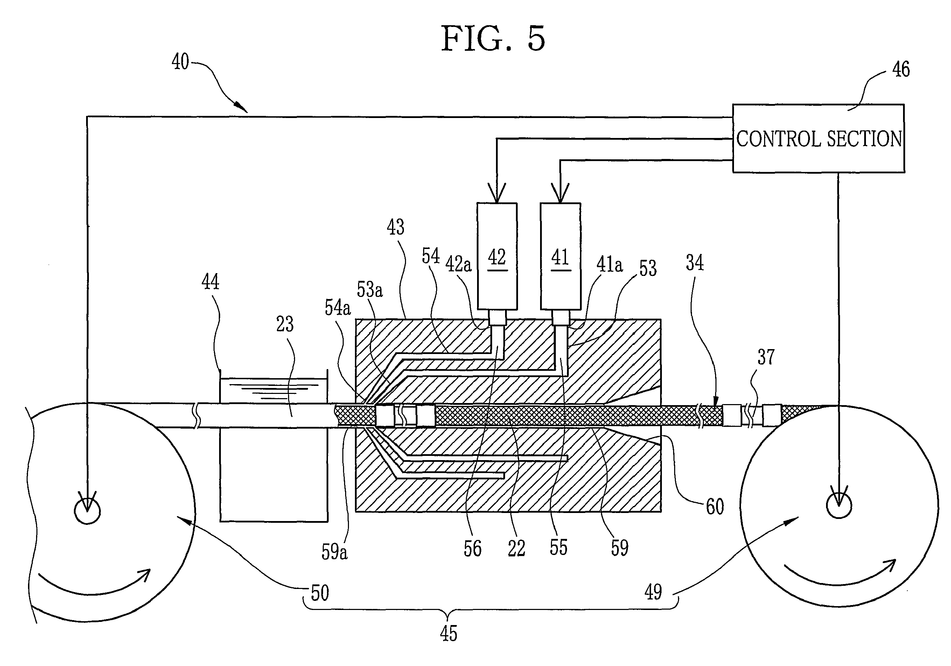

Fig. 5 shows the structure of a continuous extrusion apparatus 40 used in the resin layer forming step S1. The extrusion apparatus 40 is provided with commonly known extrusion sections 41 and 42 each of which includes a hopper, screw or the like, a head section 43 for applying the resin on the periphery of the assembly 34, a cooling section 44, an assembly feeding section 45 for feeding the assembly 34 into the head section 43, and a control section 46 for controlling the above.

The assembly feeding section 45 has a feeding drum 49 and a winding drum 50. The assembly 34 wound on the feeding drum 49 is successively pulled out and fed into the head section 43. The assembly 34 travels through the head section 43 and cooling section 44, and is wound up by the winding drum 50. The control section 46 controls the rotation of the feeding drum 49 and winding drum 50, and changes assembly feeding speed.

The extrusion section 41 supplies molten soft resin 55 into a feed pipe 53 or annular channel through a feed throat 41a. The extrusion section 42 supplies molten hard resin 56 into a feed pipe 54 or annular channel through a feed throat 42a. The control section 46 controls the extrusion pressure of the extrusion sections 41 and 42. When the assembly feeding speed is constant, controlling the extrusion pressure of the extrusion sections 41 and 42 can adjust the thicknesses of the soft resin layer 29 and hard resin layer 30. In other words, the extrusion pressure is increased to thicken the resin layer, and is decreased to thin the resin layer.

As described above, the head section 43 is provided with the feed pipes 53 and 54 through which the molten soft resin 55 and hard resin 56 is fed by the extrusion sections 41 and 42 onto the assembly 34, respectively. A circular hole 59 penetrates through the head section 43. The circular hole 59 determines the outside shape of the jacket 23 that is formed on the periphery of the assembly 34. To the circular hole 59, a feed port 53a of the feed pipe 53 and a feed port 54a of the feed pipe 54 are connected. A conical recess 60 is provided continuously from the circular hole 59 in the head section 43 to guide insertion of the assembly 34 into the head section 43.

The feed ports 53a and 54a or annular orifices of the feed pipes 53 and 54 are in the vicinity of an exit 59a of the circular hole 59. The feed port 53a is positioned upstream, and the feed port 54a is positioned downstream. Accordingly, since the soft resin 55 fed from the feed pipe 53 is first applied to the assembly 34 prior to the hard resin 56 from the feed pipe 54, the hard resin layer 30 is formed over the soft resin layer 29.

The inside diameter of the exit 59a of the circular hole 59 is equal to the outside diameter of the tubular structure 22 with the jacket 23 applied. The assembly 34 passes through the exit 59a immediately after the soft resin 55 and hard resin 56 is applied thereto from the feed ports 53a and 54a, respectively, and hence has a constant outside diameter throughout its length. The assembly 34 that has gotten out of the head section 43 is fed into the cooling section 44. In the cooling section 44, there is a tank with coolant such as water. While the assembly 34 travels through the coolant, the soft resin 55 and hard resin 56 are hardened and become the jacket 23. Instead of the above, coolant, air or the like may be sprayed to cool the resin 55 and 56.

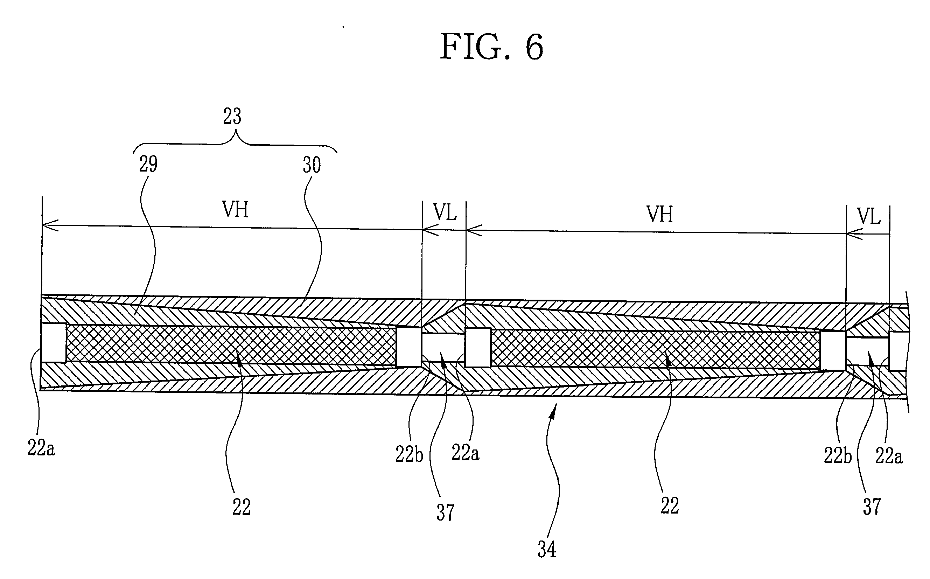

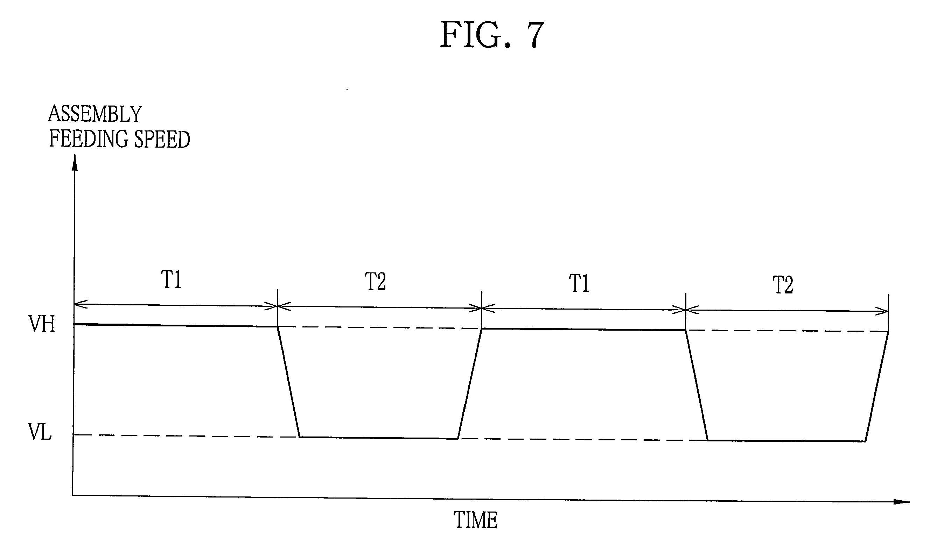

Referring to Figs. 6 and 7, the step of the extrusion apparatus 40 forming the jacket 23 on the assembly 34 will be described. Fig. 6 schematically shows variation in the thicknesses of the soft resin layer 29 and hard resin layer 30 in the resin layer forming step S1. In Fig. 6, the jacket 23 is illustrated thicker than it really is, for the sake of visual clarity. The jacket 23 is formed from left to right in Fig. 6.

In the resin layer forming step S1, the assembly feeding section 45 feeds the assembly 34 into the head section 43. At the same time, the extrusion sections 41 and 42 extrude the molten soft resin 55 and hard resin 56 into the head section 43.

From the distal end 22a to the proximal end 22b of the tubular structure 22, as shown in Fig. 6, the control section 46 controls the extrusion pressure of the extrusion sections 41 and 42, so that the soft resin layer 29 is thicker than the hard resin layer 30 at the distal end 22a, the ratio of the hard resin layer 30 is gradually increased from the distal end 22a to the proximal end 22b, and the hard resin layer 30 is thicker than the soft resin layer 29 at the proximal end 22b.

The control section 46, as shown in Fig. 7, controls the assembly feeding section 45, so as to feed the assembly 34 at a relatively high speed "VH" during a period "T1" when the resin 55 and 56 is applied on the tubular structure 22, from the distal end 22a to the proximal end 22b thereof.

In applying the resin 55 and 56 on the joint member 37 as shown in Fig. 6, on the other hand, the control section 46 controls the extrusion pressure of the extrusion sections 41 and 42, so that the hard resin layer 30 is thicker than the soft resin layer 29 in a position adjoining to the proximal end 22b of the tubular structure 22, the ratio of the soft resin layer 29 is gradually increased from the proximal end 22b to the distal end 22a of the next tubular structure 22, and the soft resin layer 29 is thicker than the hard resin layer 30 in a position adjoining to the distal end 22a. The control section 46 further controls the assembly feeding section 45 so as to feed the assembly 34 at a relatively low speed "VL" during a period "T2" (refer to Fig. 7) when the resin 55 and 56 is applied to the joint member 37. Switching the assembly feeding speed equalizes time of the period "T1" for applying the resin 55 and 56 on the long tubular structure 22 to time of the period "T2" for applying the resin 55 and 56 on the short joint member 37. For example, when the proximal end 22b of the tubular structure 22 has passed through the exit 59a of the head section 43, the assembly feeding speed is switched from "VH" to "VL". When the distal end 22a has passed through the exit 59a, the carrying speed is switched from "VL" to "VH".

When the jacket 23 is formed on the tubular structure 22, as with described above, the control section 46 controls the assembly feeding section 45 so as to switch the assembly feeding speed from "VL" to "VH", and the extrusion sections 41 and 42 so as to gradually increase the thickness of the hard resin layer 30 from the distal end 22a to the proximal end 22b. The jacket 23 is formed on the assembly 34 as described above with controlling the extrusion pressure and switching the assembly feeding speed.

In the extrusion apparatus 40, as described above, the assembly feeding speed is slowed down to "VL" in the period "T2" of applying the resin 55 and 56 to the periphery of the joint member 37. Accordingly, the extrusion pressures of the extrusion sections 41 and 42, which have been varied during applying the resin 55 and 56 on the tubular structure 22, are reset to initial values, while the resin 55 and 56 is applied to the joint member 37 and reaches the distal end 22a of the tubular structure 22 connected rearward. Thus, it is possible to continuously form the jacket 23 by laminating the soft resin layer 29 and hard resin layer 30 at a variable ratio even with the short joint member 37. Shortening the joint member 37 increases the number of flexible tubes 21 to be manufactured in predetermined time, and hence results in improvement in manufacturing efficiency and cost reduction. When the jacket 23 is formed on the joint member 37, the slow assembly feeding speed may thicken the resin layers. However, since the diameter "r" of the joint member 37 is smaller than the diameter "R" of the tubular structure 22, increase in the thickness is compensated, and the outside diameter of the jacket 23 is made constant.

The assembly 34 is cut at both ends 22a and 22b of each tubular structure 22 with the jacket 23, and the joint members 37 are detached. The jacket 23 is peeled off from the joint member 37. The joint members 37 are cleaned, and used repeatedly. Since the joint member 37 is coated with the separating material or releasing agent, as described above, it is easy to peel the jacket 23 off from the joint member 37.

The tubular structure 22 with the jacket 23 is conveyed from the extrusion apparatus 40 to a dust removing apparatus for carrying out the dust removing step S2. In the dust removing step S2, dust adhering to the surface of the hard resin layer 30 is removed. Bleed does not occur in the hard resin layer 30, as the plasticizer is not added thereto. Even if the plasticizer is added to the hard resin layer 30, the bleed hardly occurs in the hard resin layer 30 because an amount of the plasticizer added thereto is much smaller than that added to the soft resin layer 29. Thus, dust hardly adheres and is easily removed. Therefore, it is possible to reduce time and effort in the dust removing step S2, as compared with the case of providing the soft resin layer 29 outside.

The tubular structure 22 after the completion of the dust removing step S2 is conveyed to a coating apparatus for carrying out the coating step S3. In the coating step S3, the coating layer 24 is formed on the hard resin layer 30 by dip coating, spray coating or the like. The dust is removed from the surface of the hard resin layer 30 in the dust removing step S2, so that it is possible to obtain the coating layer 24 without foreign matter beneath.

Since the completed flexible tube 21 is hard on the side of the proximal end 22b, operation of the handling section 12 is efficiently transmitted to the insert section 11. The hardness is gradually reduced from the proximal end 22b to the distal end 22a, so as to improve insertability into the body cavity. Furthermore, outwardly providing the hard resin layer 30 allows increase in the volume of the hard resin layer 30, as compared with providing it inside. Thus, the hardness of the flexible tube 21 is improved without use of a hardening resin.

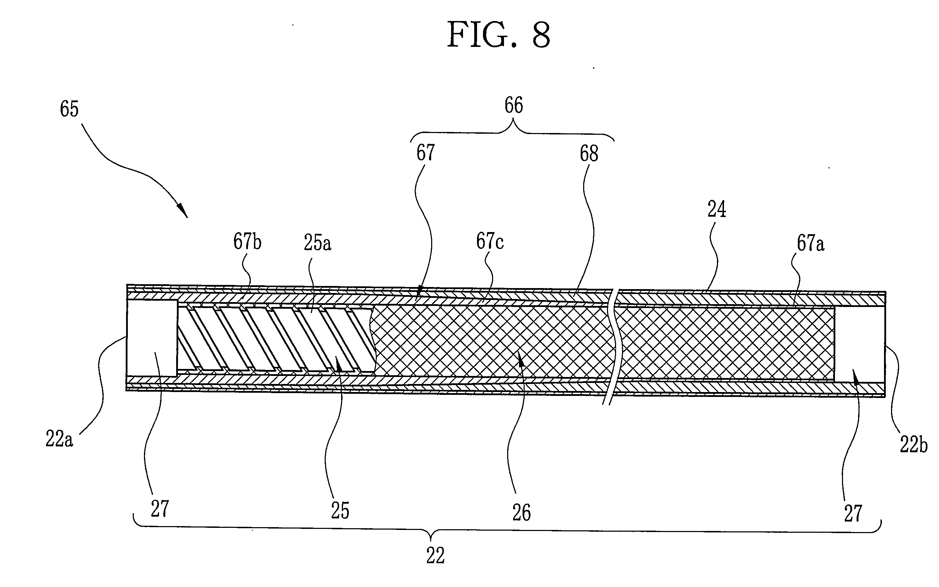

The appropriate setting of thicknesses and distribution of the hard resin layer and soft resin layer, while keeping the jacket at a constant thickness throughout the length of the tubular structure, allows variation in the hardness of a flexible tube in its longitudinal direction. For example, in a flexible tube 65 shown in Fig. 8, a soft resin layer 67 has a thin section 67a provided on the side of the proximal end 22b, a thick section 67b provided on the side of the distal end 22a, and a middle section 67c provided between the thin section 67a and thick section 67b. The thickness of the middle section 67c is varied so as to smoothly connect the thin section 67a to the thick section 67b. The thickness of a hard resin layer 68, which covers the soft resin layer 67, is thick on the side of the proximal end 22b and thin on the side of the distal end 22a. In the middle of the hard resin layer 68, the thickness thereof is gradually decreased from the proximal end 22b to the distal end 22a.

The flexible tube 65 has improved operatability because of its hardness on the side of the proximal end 22b. The flexible tube 65 also has improved insertability into the body cavity because of its softness on the side of the distal end 22a. Since the hardness of the middle portion is gradually reduced toward the distal end 22a, the handleability of the flexible tube 65 is not degraded even if the hardness is different between the proximal end 22b and distal end 22a.

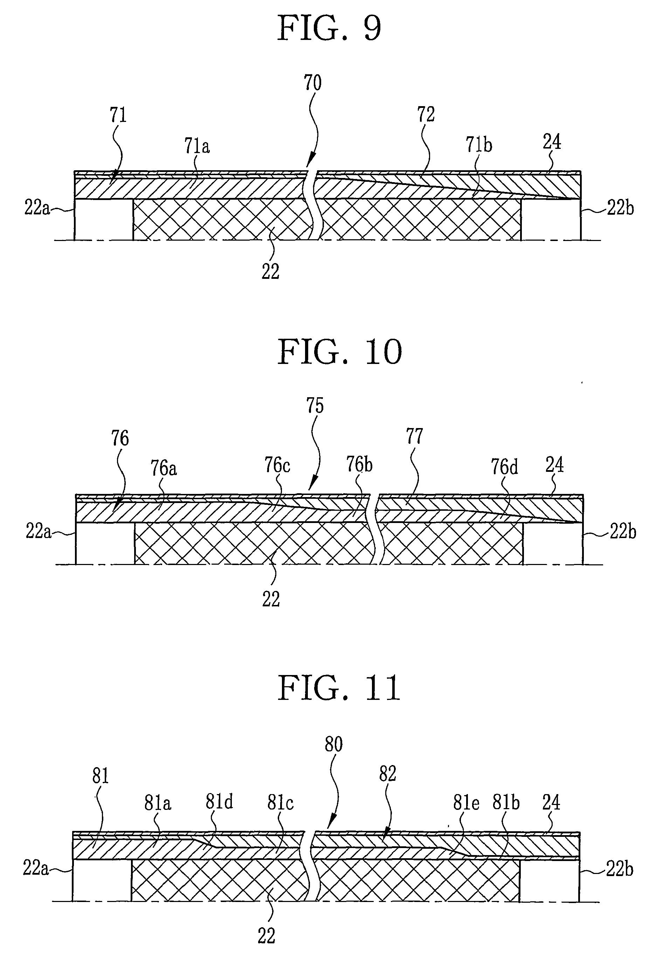

As a flexible tube 70 shown in Fig. 9, a soft resin layer 71 may have a thick section 71a provided on the side of the distal end 22a and a tapered section 71b with a decreasing thickness provided between the thick section 71a and the proximal end 22b. In this structure, the thickness of a hard resin layer 72 is decreased from the proximal end 22b to a middle portion, and is small on the side of the distal end 22a. The hardness of the flexible tube 70 is gradually reduced from the side of the proximal end 22b to the middle portion, and is low on the side of the distal end 22a.

As a flexible tube 75 shown in Fig. 10, a soft resin layer 76 may have a thick section 76a provided on the side of the distal end 22a, a medium-thickness section 76b provided in a middle portion, a connecting section 76c provided between the thick section 76a and the medium-thickness section 76b, and a tapered section 76d provided between the medium-thickness section 76b and the proximal end 22b. The thickness of the soft resin layer 76 is decreased in stages (stepwise) from the distal end 22a to the proximal end 22b, and the thickness of a hard resin layer 77 is increased conversely. Accordingly, the hardness of the flexible tube 75 is lowered in stages from the proximal end 22b to the distal end 22a.

As a flexible tube 80 shown in Fig. 11, a soft resin layer 81 may have a thick section 81a provided on the side of the distal end 22a, a thin section 81b provided on the side of the proximal end 22b, and a medium-thickness section 81c provided in a middle portion. A connecting section 81d connects the thick section 81a and the medium-thickness section 81c. A connecting section 81e connects the medium-thickness section 81c and the thin section 81b. The thickness of the soft resin layer 81 is decreased in stages (stepwise) from the distal end 22a to the proximal end 22b, and the thickness of a hard resin layer 82 is increased conversely. Therefore, the hardness of the flexible tube 80 is high on the side of the proximal end 22b, low on the side of the distal end 22a, and medium in the middle portion.

Although the present invention has been fully described by the way of the preferred embodiment thereof with reference to the accompanying drawings, various changes and modifications will be apparent to those having skill in this field. Therefore, unless otherwise these changes and modifications depart from the scope of the present invention, they should be construed as included therein.

## Claims

1. A flexible tube (21) comprising:
a flexible tubular structure (22);
a jacket (23) for covering the periphery of said tubular structure with a predetermined thickness, said jacket having a soft resin layer (29) applied on said tubular structure and a hard resin layer (30) applied on said soft resin layer; and
a coating layer (24) for coating said jacket.

2. The flexible tube (21) according to claim 1, wherein said tubular structure (22) has a first end (22a) and a second end (22b).

3. The flexible tube (21) according to claim 2, wherein said soft resin layer (29) is thicker at said first end (22a) of said tubular structure (22) than at said second end (22b) thereof.

4. The flexible tube (21) according to claim 2, wherein the thickness of said soft resin layer (29) continuously varies from said first end (22a) to said second end (22b) of said tubular structure (22).

5. The flexible tube (21) according to claim 2, wherein the thickness of said soft resin layer (29) varies in stages from said first end (22a) to said second end (22b) of said tubular structure (22).

6. The flexible tube (65) according to claim 5, wherein said soft resin layer (67) comprises:
a thick section (67b) provided on the side of said first end (22a);
a thin section (67a) provided on the side of said second end (22b); and
a middle section (67c) provided between said thick section and said thin section, the thickness of said middle section continuously decreasing from said thick section to said thin section.

7. The flexible tube (70) according to claim 5, wherein said soft resin layer (71) comprises:
a thick section (71a) provided on the side of said first end (22a); and
a tapered section (71b) provided between said thick section and said second end (22b), the thickness of said tapered section continuously decreasing from said thick section to said second end.

8. The flexible tube (75) according to claim 5, wherein said soft resin layer (76) comprises:
a thick section (76a) provided on the side of said first end (22a);
a medium-thickness section (76b) provided in the middle of said tubular structure (22);
a connecting section (76c) provided between said thick section and said medium-thickness section, for connecting said thick section to said medium-thickness section by continuously decreasing thickness; and
a tapered section (76d) provided between said medium-thickness section and said second end (22b), the thickness of said tapered section continuously decreasing from said medium-thickness section to said second end.

9. The flexible tube (80) according to claim 5, wherein said soft resin layer (81) comprises:
a thick section (81a) provided on the side of said first end (22a);
a medium-thickness section (81c) provided in the middle of said tubular structure (22);
a thin section (81b) provided on the side of said second end (22b);
a first connecting section (81d) provided between said thick section and said medium-thickness section, for connecting said thick section to said medium-thickness section by continuously decreasing thickness; and
a second connecting section (81e) provided between said medium-thickness section and said thin section, for connecting said medium-thickness section to said thin section by continuously decreasing thickness.

10. The flexible tube (21) according to claim 4, wherein the thickness of said soft resin layer (29) continuously decreases from said first end (22a) to said second end (22b), the thickness of said hard resin layer (30) continuously increases from said first end to said second end, and the outside diameter of said jacket (23) for covering said tubular structure (22) is substantially constant throughout its length.

11. The flexible tube (21) according to claim 5, wherein the thickness of said soft resin layer (29) decreases in stages from said first end (22a) to said second end (22b), the thickness of said hard resin layer (30) increases in stages from said first end to said second end, and the outside diameter of said jacket (23) for covering said tubular structure (22) is substantially constant throughout its length.

12. An endoscope (10) having the flexible tube (21) according to claim 1.

13. A method for manufacturing a flexible tube (21) comprising the steps of:
feeding a flexible tubular structure (22) into a head section (43), and passing said tubular structure through said head section;
extruding soft resin in said head section to form a soft resin layer (29) on the periphery of said tubular structure;
extruding hard resin in said head section to form a hard resin layer (30) on said soft resin layer;
adjusting extrusion pressure of said soft resin in accordance with the thickness of said soft resin layer, and adjusting extrusion pressure of said hard resin in accordance with the thickness of said hard resin layer;
cooling said tubular structure with a jacket (23) containing two layers of said soft resin layer and said hard resin layer; and
winding up said tubular structure with said jacket.
